**Europäisches Patentamt**

(19) **European Patent Office**

**Office européen des brevets**

(11) Veröffentlichungsnummer : **0 139 913 B2**

(12) # NEUE EUROPÄISCHE PATENTSCHRIFT

(45) Veröffentlichungstag der neuen Patentschrift :
29.01.92 Patentblatt 92/05

(51) Int. Cl.$^5$ : **C08L 5/14, A23L 1/0522**

(21) Anmeldenummer : **84109242.2**

(22) Anmeldetag : **03.08.84**

(54) Gelier- und Verdickungsmittel auf der Basis von Cassia-Galactomannanen.

(30) Priorität : **30.09.83 DE 3335593**

(43) Veröffentlichungstag der Anmeldung :
08.05.85 Patentblatt 85/19

(45) Bekanntmachung des Hinweises auf die
Patenterteilung :
10.12.86 Patentblatt 86/50

(45) Bekanntmachung des Hinweises auf die
Entscheidung über den Einspruch :
29.01.92 Patentblatt 92/05

(84) Benannte Vertragsstaaten :
AT BE CH DE FR GB IT LI LU NL SE

(56) Entgegenhaltungen :
EP-A- 0 018 153
DD-A- 121 707
DE-A- 2 836 140
US-A- 3 367 783
Patent Abstracts of Japan Band 7, Nr. 201,
6.9.83, Seite C184 (1346)
Robertson & Lee "Journal of the Arnold Arboretum" Bd. 57 no. 1 Jan. 1976 S. 35-46
Whistler "Industrial Gums" 2 Ausgabe 1973 S.
330-337
Dea & Morrison. "Chemistry & Interactions of
seed Galactomannans" Advances in Carbohydrate Chemistry & Biochemistry Bd. 31 1975
S. 241-312
Hui. "Untersuchung an Galaktomannanen"
nr. 3297, 1962
Industrial & Engineering chemistry Bd. 41 1949
S: 2888
Indian Journal of Chemistry Bd. 11 1973 S: 505
"Carbohydrate Research " Bd. 71, 1979
p.205ff
"Carrageenan" Broschüre der firma Marine
Colloids Inc
Chimia Band 8 1954 S. 64-79
"Xanthan Gum" Broschüre der Firma Kelco
1975
"Hamulsion DS" Merkblatt der firma Hahn &
Co 1 Abschnitt.
"Stabilisation of cottage cheese dressing with
GFS" Merkblatt der fa. Kelco 1973
Handbook of water-soluble gums & resins, Mc
Graw Hill, 1980

(56) Entgegenhaltungen :
"Verdickungsmittel und Kleber für den Textildruck und verwandte Arbeitsgebiete" Bayern
Farben Revue Sonderdruck 1981
"Carbohydrate research" 92 1981 s. 269-285
"Industrial gums, polysaccharides and their
derivatives" Whistler & Bemiller, Academie
Pres s, 1959. S. 106-109
Owens & Lewis Pl. Syst Evol 163, pp 93-105,
1989
Prog. Fd. Nutr. Science 6 1982. s. 109-118
Phytochemistry 15, 1976, s. 1111-1117. McCleary et al
Journal of mol. Biology 68, 1972 p. 153-172.
Dea, McKinnon & Rees
Economic Botany 19 1965.p. 165-173. Tockey &
Jones
Handbook of food additives 2nd. edition. Furia,
1975p. 327

(73) Patentinhaber : **DIAMALT
AKTIENGESELLSCHAFT
Georg-Reismüller-Strasse 34
W-8000 München 50 (DE)**

(72) Erfinder : **Bayerlein, Friedrich, Dr.
Schwalbenweg 7
W-8033 Krailling (DE)**
Erfinder : **Kuhn, Manfred, Dr.
Hornstrasse 22
W-8000 München 40 (DE)**
Erfinder : **Maton, Michel
39, Avenue Clarisse
F-92420 Vaucresson (FR)**

(74) Vertreter : **Lederer, Franz, Dr. et al
Lederer, Keller & Riederer Patentanwälte
Lucile-Grahn-Strasse 22
W-8000 München 80 (DE)**

EP 0 139 913 B2

## Beschreibung

Unter Gelier- und Verdickungsmitteln versteht man Stoffe, die beispielsweise Wasser oder wässerigen Behandlungsflüssigkeiten oder festen oder flüssigen Lebens- bzw. Futtermitteln während des Herstellungs- und Verarbeitungsprozesses zugesetzt werden, um eine gewünschte Konsistenz oder Viskosität zu erzielen. Nahezu alle gebräuchlichen Gelier- und Verdickungsmittel, mit Ausnahme der Gelatine, sind Abkömmlinge der Polysaccharide, d.h. von hochpolymeren Kohlehydraten.

Polysaccharide sind wasserlösliche oder stark quellbare Stoffe, die sog. Hydrokolloide, die in wässerigen Systemen kolloidale, mehr oder weniger hochviskose Lösungen oder Dispersionen mit plastischem oder pseudoplastischem Fliessverhalten ergeben. Daraus leiten sich die im vorliegenden Fall gewünschten funktionellen Eigenschaften wie verdickende Wirkung, Wasserbindevermögen, Stabilisierung von Suspensionen und Emulsionen bei mehrphasigen Systemen sowie Gelbildung ab.

Galactomannane sind, wie die Stärke, pflanzliche Reservepolysaccharide, die in den Endospermzellen vieler Leguminosensamen vorkommen. Bei der Keimung der Samen werden sie enzymatisch abgebaut und dienen als Nährstoffe für den Keimling. Der Sammelbegriff Galactomannan bzw. Polygalactomannan umfasst alle Polysaccharide, die aus Galactose- bzw. Mannosebausteinen aufgebaut sind und darüberhinaus in untergeordnetem Masse auch weitere Zuckerbausteine aufweisen können. Ja nach Herkunft gibt es eine verhältnismässig grosse Anzahl von Galactomannanen. Sie kommen hauptsächlich in den Endospermabschnitten und Samen verschiedener Leguminosen (Hülsenfrüchtlern) wie Guar, Johannisbrot, Tara, Honigbohne, Flammenbaum, Sesbania und Cassia-Arten vor. Galactomannane sind aufgebaut aus einer linearen Mannosekette, die aus β-(1,4)-glucosidisch verknüpften Mannopyranoseringen aufgebaut ist. An diese sind als Verzweigung einzelne Galactopyranosereste in α-(1,6)-glucosidischer Bindung fixiert.

Von den zahlreichen bekannten Galactomannanen werden insbesondere drei gewonnen und verwendet:

1. *Johannisbrotkernmehl* (Carubin, Locust Bean Gum) ist seit langem bekannt. Es wird aus dem Samen des Johannisbrotbaumes (Ceratonia siliqua L.) gewonnen, der in den Mittelmeerländern beheimatet ist.

2. *Guargummi* (Guaran, Guar Gum) ist heute das wichtigste Galactomannan. Es wird aus den Samen der in Indien und Pakistan beheimateten Guarbohne (Cyamopsis tetragonolobus L. taub.) gewonnen.

3. *Taragummi* (Tara Gum) wird erst seit kurzem in geringen Mengen aus den Samen des Tarabaumes (Cesalpinia spinosa) produziert, der vor allem in Peru wächst.

Carrageenane und Agar sind Extrakte aus Rotalgen und gehören chemisch zur Gruppe der Galactane. Sie weisen jedoch nicht, wie Cellulose und Stärke, nur einen 1,4-Glycosid-Bindungstyp auf. Die Rotalgen-Galactomannane besitzen vielmehr abwechselnd α-1,3-Bindungen und β-1,4-Bindungen und werden deshalb als ab-a-Typ Polysaccharide charakterisiert. Carrageenan ist chemisch kein homogenes Produkt, sondern umfasst die Produktgruppe von sulfatierten Galactanen, wobei Teile der Galactopyranosebausteine als 3,6-Anhydrogalactose-Baustein vorliegen. Aus den Rotalgenextrakten lassen sich bestimmte Fraktionen von Carrageenan gewinnen, die chemisch durch ihre Struktur definiert sind und mit griechischen Buchstaben bezeichnet werden. Kommerzielle Bedeutung haben nur Lambda-, Jota- und Kappa-Carrageenan. Die unterschiedlichen Eigenschaften finden ihre Erklärung hauptsächlich durch Unterschiede im Gehalt an Anhydrogalactose und Sulfatestergruppen. Durch den 3,6-Anhydrogalactosering werden die Galactane stärker hydrophobiert, d.h. die Wasserlöslichkeit nimmt ab.

Andererseits verleiht die Sulfatgruppe den Galactanen mehr hydrophile Eigenschaften, d.h. die Wasserlöslichkeit nimmt zu. Weiterhin hat das Vorliegen der Sulfatgruppen zur Folge, dass sich die Eigenschaften von Carrageenan als anionisches Polysaccharid durch das Vorliegen von Kationen im wässerigen System verändern lassen. So werden die Geliereigenschaften von Kappa-Carrageenan durch Kalium-Ionen und von Jota-Carrageenan durch Calcium-Ionen stark beeinflusst.

Bei Agar, einem elektroneutralen Galactan mit einem hohen Anhydrogalactosegehalt, verläuft die Gelierung hingegen unabhängig von Kationen. Von den Carrageenanen weist das Kappa-Carrageenan den höchsten Anhydrogalactosegehalt und den niedrigsten Sulfatgehalt auf und erhält hierdurch die stärksten Gelbildungseigenschaften mit der schon erwähnten grossen Abhängigkeit von der Kalium-Ionen-Konzentration.

Lambda-Carrageenan hingegen enthält keine Anhydrogalactose und den grössten Sulfatestergehalt unter den Carrageenanen. Dies hat zur Folge, dass es nicht mehr zum Gelieren gebracht werden kann. Ein Formelbild mit der idealisierten Struktur von Kappa-Carrageenan findet sich bei Robert L. Davidson "Handbook of Water-Soluble Gums and Resins", Figur 5.2, Mc Graw-Hill Book Company (1980).

Xanthan ist ein hochmolekulares Polysaccharid, das in einem Fermentationsprozess durch die Mikroorganismen Xanthomonas campestris gewonnen wird. Die Hauptkette des Xanthans hat Cellulosestruktur. Sie besteht aus D-Glucoseeinheiten mit β-1,4-Bindungen. Die Trisaccharidseitenketten bestehen aus 2 Mannoseeinheiten und 1 Glucuronsäureeinheit. Die endständige β-D-Mannoseeinheit ist glykosidisch verknüpft mit der 4-Position der β-D-Glucuronsäure, die ihrerseits glykosidisch verknüpft ist mit der 2-Position der α-D-Mannose. Diese Seitenkette ist mit der 3-Position eines jeden zweiten Glucoserestes der Polymerhauptkette verknüpft. Ungefähr die Hälfte der endständigen D-Mannosereste tragen einen Brenztraubensäurerest der ketalisch mit den 4- und 6-Positionen des Mannoseringes verknüpft ist. Die nicht-endständige D-Mannoseeinheit der Seitenkette trägt eine Acetylgruppe in der 6-Stellung. Die Glucuronsäuregruppe liegt als gemischtes Kalium-, Natrium- und Calcium-Salz vor. Ein Ausschnitt aus der Xanthanpolymerkette findet sich bei Robert L. Davidson "Handbook of Water-Soluble Gums and Resins", Figur 24.1, Mc Graw-Hill Book Company (1980).

Obwohl Carrageenan-Wassergele schon sehr lange bekannt waren, fanden sie lange Zeit keine Anwendung, weil sie unerwünschte spröde und kohesive Eigenschaften hatten und deshalb den normalerweise eingesetzten, hochelastischen Pektin- und Gelatinegelen unterlegen waren. Dies änderte sich dann mit der nun auch schon über 30 Jahre lange bekannten Entdeckung, dass sich durch Einbau eines neutralen Polymers, in diesem Falle Johannisbrotkernmehl, die Sprödigkeit und Steifheit von reinen Carrageenangelen zu einem elastischen Gel modifizieren liess. Diesen synergistischen Effekt mit Johannisbrotkernmehl zeigen auch Agar, Kappa-Carrageenan und Xanthan. Bei Agar und besonders Kappa-Carrageenan äussert sich dies in höherer Gelstärke und Elastizität der Gele. Mit Xanthan, das für sich allein kein Geliermittel ist, bildet Johannisbrotkernmehl thermoreversible Gele mit grosser Kohäsion bzw. sehr viskosen Lösungen bei extrem niedrigen Konzentrationen.

Die anderen erwähnten Galactomannane, nämlich Guar Gum und Tara Gum zeigen diesen synergistischen Effekt nicht oder nur in stark abgeschwächtem Ausmass. Dies ist um so erstaunlicher als alle drei erwähnten Polysaccharide der Gruppe der Galactomannane angehören und sich voneinander nur durch das unterschiedliche Verhältnis Galactose: Mannose unterscheiden. So trägt z.B. im Guaran jeder zweite Mannopyranosering der Hauptkette einen Galactopyranosering. Das entspricht einem Galactosegehalt von 33-34% und einem Mannosegehalt von 66-67%. Im Tara-Galactomannan ist nur an jede dritte Mannopyranoseeinheit der Hauptkette eine Galactopyranoseeinheit geknüpft. Hieraus resultiert ein Galactose: Mannose-Verhältnis von 25:75.

Johannisbrotkernmehl ist ein Galactomannan mit einer Mannose-Hauptkette, in der durchschnittlich jede vierte Mannopyranoseeinheit mit einem Galactoserest substituiert ist. Obwohl das durchschnittliche Verhältnis Galactose: Mannose ungefähr 1:4 ist, sind die Galactoseseitengruppen oft so angeordnet, dass im Mannose-Grundgerüst Zonen mit fortlaufender Galactosesubstitution entstehen, d.h. Regionen, in denen jede Mannopyranosyleinheit mit einem Galactoserest substituiert ist und Zonen mit relativ langen Abschnitten des unsubstituierten Mannosegerüstes.

Nahrungsmittelgele zeigen viskoelastische Eigenschaften. Ob nun die elastische (feste) oder viskose (flüssige) Komponente überwiegt, hängt von den Kräften ab, die auf das rheologische System einwirken und folglich von dem Ausmass, wie die verknüpfte Netzwerkstruktur beschädigt wird. Um die elastischen Eigenschaften eines Gels zu charakterisieren, bedient man sich in der Praxis verschiedener Messmethoden bzw. Messungen. Man unterscheidet zwischen Messmethoden, bei denen die elastischen Grenzen des Gels überschritten werden und das Gel bricht und Messmethoden, bei denen nur die elastische Deformation von Gelen ohne Überschreiten der Elastizitätsgrenzen gemessen wird. Zu dieser Gruppe gehören Geräte wie das Bloom-Gelometer, B.A.R.-Jellytester. Exchange Ridgelimeter, F.I.R.A. Jellytester, Cox und Higby SAG-Methode und Abwandlungen hiervon, Saverborns Zylindrische Torsionsmethode.

Im Rahmen der vorliegenden Erfindung wurden für die Gelmessungen der F.I.R.A. Jellytester und für Viskositätsmessungen das Brookfield Rotationsviskosimeter RVT verwandt. Der F.I.R.A. Jellytester besteht im wesentlichen aus einem schmalen Metallblatt, das auf einen Schaft montiert ist, der eine genaue und leicht ablesbare, von -10 bis +90 Winkelgrad kalibrierte Skala trägt. Diese ganze Einrichtung ist beim Auftreten einer Torsionskraft rotierbar; die Torsionskraft wird durch fliessendes Wasser erzeugt, das mit einer vorgegebenen Geschwindigkeitsrate in einen kleinen, mit Gegengewicht versehenen Behälter, der über eine Zugvorrichtung mit dem Schaft verbunden ist, läuft. Die Gelstärke wird nun gemessen, indem man das Metallblatt in das Gel eintaucht und solange Wasser in den kleinen Behälter fliessen lässt bis das Metallblatt um einen bestimmten Winkel rotiert. Je höher nun die zum Erreichen des vorgegebenen Deformationswinkels benötigte Wassermenge ist, desto höher ist die Gelstärke. In den angeführten Beispielen beträgt der Deformationswinkel 30° und die hierfür benötigte Wassermenge wird in ml angegeben.

Erfindungsgemäss wurde nun gefunden, dass ein aus den Endospermabschnitten von Cassia-tora Samen gewonnenes Galactomannanmehl im Gegensatz zu Guar- und Taramehl einen starken Synergismus mit den Rotalgenextrakten Carrageenan und Agar sowie mit dem Biopolymer Xanthan aufweist, der ausserdem wesentlich stärker ist als der Synergismus den Johannisbrotkernmehl mit diesen Polysacchariden zeigt. Dies war völlig überraschend, da men bisher annahm, dass Johannisbrotkernmehl bezüglich des synergistischen Effektes eine Sonderstellung unter den Galactomannanen einnimmt.

Gegenstand der vorliegenden Erfindung ist daher ein Gelier- und Verdickungsmittel auf der Basis von Cassia-Galactomannenen, welches eine synergistische Mischung aus a) Cassia-tora Galactomannan und b) Carrageenan, Agar und/oder Xanthan enthält oder aus einer solchen Mischung besteht.

Das erfindungsgemäss eingesetzte Cassia-Galactomannan ist ein solches, das der botanischen Art Cassia tora (L. Baker) bzw. deren Synonyma Cassia obtusifolia (Linn.) bzw. Cassia toroides entstammt.

Die Komponenten a), also das Cassia-Galactomannan, und b), also das Carrageenan, Agar und/oder Xanthan, liegen im allgemeinen in einem Gewichtsverhältnis von a:b = (10-90) : (90-10), vorzugsweise in einem Gewichtsverhältnis von a:b = (40-60) : (60-40) vor.

Zur Verbesserung der Gelbildungseigenschaften kann das erfindungsgemässe Gelier- und Verdickungsmittel noch Kalium-, Calcium- und/oder Ammoniumionen enthalten. Insbesondere bei Vorliegen von Carrageenan ist ein solcher Elektrolytzusatz vorteilhaft. So kann, insbesondere bei Anwesenheit von Kappa-Carrageenan, ein Zusatz von 1-50, vorzugsweise 10-40 Gew.-% Kaliumchlorid bezogen auf die Komponente b) vorteilhaft sein. Das erfindungsgemässe Gelier- und Verdikkungsmittel kann in Form einer pulverförmigen Mischung vorliegen und ist so am besten handhabbar.

In Wasser erhitzt tritt die Gelierung ein und das Mittel kann auch in angelierter oder vollständig gelierter Form, mit geringerem oder höherem Wassergehalt vorliegen.

Die erfindungsgemässen Gelier- und Verdikkungsmittel sind auf all den Gebieten mit Vorteil einsetzbar, wo man die wasserverdickende Wirkung von Galactomannan, Carrageenan, Agar-Agar sowie Xanthan benötigt. Typische Einsatzgebiete sind beispielsweise: Futtermittel (Tierfutter), Nahrungsmittel, als Flockungs-, Absetz- und Filterhilfsmittel, auf den Gebieten Bergbau und Wasserbehandlung, Verdickungsmittel für pharmazeutische und kosmetische Zwecke, Additive für die Papierherstellung, Zusätze bei der Erdöl- und Wasserbohrung, Sprengstofformulierungen, Wasserretentionsmittel z.B. in Baustoffen, Verdickungsmittel z.B. für textile Anwendungen wie Druckpasten. Kleber und Schlichten, Tabakbindemittel und viele weitere Einsatzgebiete, wo sich der Fachmann die verdickenden, gelierenden, suspendierenden, emulgierenden, stabilisierenden, schmierenden, filmbildenden und bindenden Eigenschaften solcher Verdickungs- und Geliermittelsysteme zunutze macht. Futtermittel- und Nahrungsmittelgele mit den erfindungsgemässen Geliermitteln zeigen häufig bessere organoleptische Eigenschaften als Gele, die nur aus Carrageenan oder Agar bestehen.

Die in den Beispielen genannten Teile sind Gewichtsteile.

## Beispiel 1:

In diesem Beispiel wird der Unterschied im synergistischen Verhalten bei der Gelbildung mit Kappa-Carrageenan zwischen den verschiedenen Galactomannanen von Guar, Tara, JBK und Cassia demonstriert. Es wurden 1:1 Mischungen aus Galactomannan und kommerziell erhältlichem Kappa-Carrageenan (Danagel CCX) hergestellt. Von diesen Geliermittelmischungen wurden jeweils 4 Teile in 1000 Teile Wasser bei Raumtemperatur mit einem Schnellrührer eingerührt. Anschliessend wurde die Mischung im Becherglas unter leichtem Rühren auf 85° C 5 Minuten lang erhitzt und der beim Erhitzen entstandene Wasserverlust kurz vor dem Abkühlen durch Hinzufügen von heissem Wasser wieder ausgeglichen. Die noch heissen, leicht viskosen Lösungen wurden nun in die zum F.I.R.A. Jellytester gehörenden Messbecher gegossen. Nach dem Abkühlen auf 23° C im Thermostaten wurden am nächsten Tag die Gelstärken in Form der Anzahl an ml Wasser gemessen, die notwendig waren, die Skala am Testgerät um 30° auszulenken. Diese Verfahrensweise wurde bei allen Untersuchungen mit dem F.I.R.A. Jellytester beibehalten.

| Mischung | Gelstärke |
|---|---|
| Guar/Carrageenan | keine Gelbildung |
| Tara/Carrageenan | keine Gelbildung |
| JBK/Carrageenan | kaum Gelbildung, nicht messbar |
| Cassia/Carrageenan | 8 |

## Beispiel 2:

In diesem Beispiel wurde die Menge an Geliermittelmischungen gemäss Beispiel 1 von 4 Teile auf 6 Teile/1000 Teile erhöht, da in Beispiel 1 mit JBK-Mehl noch keine messbare Gelbildung erreicht wurde.

| Mischung | Gelstärke |
|---|---|
| Guar/Carrageenan<br>Tara/Carrageenan | keine Gelbildung<br>geringfügige<br>Gelbildung,<br>nicht messbar |
| JBK/Carrageenan | 25 |
| Cassia/Carrageenan | 33 |

Das Ergebnis zeigt, dass der Austausch von JBK-Galactomannan gegen Cassia-Galactomannan zu einer stärkeren Gelierung führt. Die Gelstärke liegt im System Cassia/Kappa-Carrageenan um ca. 30% höher als im System JBK/Carrageenan. Die anderen beiden Galactomannane aus Guar bzw. Tara führten zu keiner bzw. mit dem F.I.R.A. Jellytester nicht messbaren Gelbildung.

*Beispiel 3:*

Dieses Beispiel erläutert den Einfluss von Kalium-Ionen auf das erfindungsgemässe Gelsystem. Kommerziell erhältliche Kappa-Carrageenane liegen in Form von Mischsalzen vor und enthalten durchschnittlich 3,5% Calcium, 0,1% Magnesium, 1,5% Kalium und 1,5% Natrium. In der reinen Natriumform gelieren Kappa-Carrageenane nicht. In der Praxis setzt man deshalb zur Verstärkung der Gelbildung häufig Kalium-, Calcium- und Ammonium-Ionen zu, wobei beim Kappa-Carrageenan durch Kalium-Ionen die stärksten Gele erzeugt werden. Typische Mengenzusätze an Kaliumchlorid bewegen sich je nach Anwendungszweck bis zu 50% des eingesetzten Kappa-Carrageenan-Anteils, d.h. dass in wässerigen Systemen Zusätze bis zu 3 g/l Kaliumchlorid durchaus praxisüblich sind. Am F.I.R.A. Jellytester wurden 1:1-Mischungen aus kommerziell erhältlichem JBK-Mehl mit Kappa-Carrageenan (Danagel CCX) sowie Cassia-Galactomannanmehl hergestellt. Es wurden wie im 1. Beispiel 4 Teile Geliermittelmischung auf 1000 Teile Wasser eingewogen, wobei das Wasser zusätzlich 1 g/l Kaliumchlorid enthielt. In einem 2. Versuch wurde die Menge an Geliermittelmischung auf 6 Teile/1000 Teile Wasser erhöht, wobei die Konzentration an Kalium-Ionen konstant gehalten wurde.

| Mischung | Gelstärke<br>4 Teile/1000<br>Teile Wasser | Gelstärke<br>6 Teile/1000<br>Teile Wasser |
|---|---|---|
| JBK/Carrageenan | 36 | 71 |
| Cassia/<br>Carrageenan | 44 | 80 |

Dieses Beispiel macht deutlich, dass bei einem Zusatz von 0,1 % Kaliumchlorid zum Gel bei einem Einsatz von 4 g/l Geliermittelmischung immer noch eine Steigerung der Gelstärke beim Ersatz von JBK-Mehl durch Cassia-Mehl von über 20% resultiert. Bei einem Einsatz von 6 g/l Geliermittelmischung beträgt der Vorteil immer noch über 10%.

*Beispiel 4:*

In Abänderung zu Beispiel 3 wurde die Konzentration von Kaliumchlorid im Gel auf 2 g/l entsprechend 0,2% erhöht. Im übrigen wurde wie in Beispiel 3 verfahren.

| Mischung | Gelstärke<br>4 Teile/1000<br>Teile Wasser | Gelstärke<br>6 Teile/1000<br>Teile Wasser |
|---|---|---|
| JBK/Carrageenan | 44 | 76 |
| Cassia/<br>Carrageenan | 49 | 82 |

Auch bei der von 1 g/l auf 2 g/l erhöhten Einsatzmenge von Kaliumchlorid ändert sich an den in Beispiel 3 erläuterten Verhältnissen nichts. Bei einer Anwendungsmenge von 4 g/l Geliermittelmischung beträgt der Vorteil von Cassia gegenüber JBK über 10%. Bei einer auf 6 g/l erhöhten Gelier- mittelmenge beträgt der Nachteil von JBK gegenüber Cassia noch etwa 8%.

*Beispiel 5:*

Im Gegensatz zu den in Beispiel 3 genannten Kationen bewirken Natrium-Ionen eine Schwächung der Gelnetzstruktur und führen zu niedrigeren Gelstärken. Es wurden wieder 1:1 Mischungen JBK/Carrageenan bzw. Cassia/Carrageenan hergestellt und aus diesen in der bekannten Weise Gele erzeugt, die zusätzlich noch 0,5, 1 und 3% Natriumchlorid enthielten.

| Mischung 6 Teile/1000 Teile Wasser | 0,5% NaCl | 1% NaCl | 3% NaCl |
|---|---|---|---|
| JBK/Carrageenan | 21 | 15 | 7 |
| Cassia/Carrageenan | 43 | 31 | 12 |

Auch Gel-schwächende Elektrolyte wie Natriumchlorid ändern aber nichts an dem in den Beispielen 1-4 aufgezeigten überlegenen Verhalten des Cassia-Galactomannanmehles gegenüber JBK-Galactomannanmehl.

Bei allen drei im Beispiel genannten Natriumchloridkonzentrationsbereichen ergibt sich für Cassia eine Überlegenheit in der Gelstärke gegenüber dem JBK-Mehl von ungefähr 50%.

*Beispiel 6:*

In diesem Beispiel wird das System Galactomannan/Xanthan beschrieben und die synergistischen Wirkungen der einzelnen Galactomannane mit dem Biopolymer Xanthan verglichen. Die rheologischen Eigenschaften sind vor allem dadurch charakterisiert, dass Xanthane extrem pseudoplastisches Fliessverhalten aufweisen, ohne jedoch eigentlich Gele zu bilden. D.h. nach Überwinden der Fliessgrenze geht beim Auftreten von Schubspannung die Viskosität proportional zur Scherbelastung zurück. Die ursprüngliche Viskosität bildet sich fast unmittelbar nach dem Ende der Scherbelastung wieder aus. Die nachfolgende Tabelle zeigt die Kalt- und Heissviskositäten von Guar-, JBK- und Tara- und Cassia tora L. -Galactomannan sowie Xanthan. Es handelt sich jeweils um 1%ige wässerige Lösungen, gemessen am Brookfield RVT bei 20°C und bei 20 UpM. Die Viskositätsmessungen erfolgten, nachdem die Polymerpulver während 20 Minuten klumpenfrei an einem Schnellrührsystem eingerührt worden waren und nach einer Standzeit von insgesamt 2 Stunden. Unter Heissviskosität versteht man den Viskositätswert, der ebenfalls nach 20minütigem klumpenfreien Einrühren des Polymerpulvers, anschliessendem 5minütigem Erhitzen auf ca. 90° C, Ausgleich des hierbei entstandenen Wasserverlustes und Abkühlen auf 20° C gemessen wurde.

| | Heissviskosität | Kaltviskosität |
|---|---|---|
| Cassia tora L. | 260 mPas | unmessbar dünn |
| JBK | 2 450 mPas | 78 mPas |
| Tara | 3 600 mPas | 3 040 mPas |
| Guar | 5 300 mPas | 5 100 mPas |
| Xanthan (Rhodigel 23) | 3 200 mPas | 3 200 mPas |

Es wurden nun 1:1-Mischungen aus Galactomannan und Xanthan hergestellt, 1%ig in Wasser eingerührt, auf 90° erhitzt und nach Ausgleich des Wasserverlustes auf 20° abgekühlt.

Die Mischung Guar/Xanthan zeigte keinerlei Gelbildung, die Mischung Tara: Xanthan minimale Gelbildung. Die stärksten Geliereigenschaften zeigte das System Cassia tora L./Xanthan. Die Ergebnisse sind in der Tabelle festgehalten.

| Mischung | Gelstärke |
|---|---|
| Guar/Xanthan | keine Gelbildung |
| Tara/Xanthan | 2 |
| JBK/Xanthan | 4 |
| Cassia tora L./ Xanthan | 11 |

Die Gelstärken wurden wiederum am F.I.R.A. Jellytester gemessen, der Auslenkwinkel betrug wie in allen anderen vorhergehenden Fällen 30°.

## Patentansprüche

### Patentansprüche für folgende Vertragsstaaten : BE, CH, LI, FR, GB, IT, LU, NL, SE

1. Gelier- und Verdickungsmittel auf der Basis von Cassia-Galactomannanen dadurch gekennzeichnet, dass eine synergistische Mischung aus a) Cassia-tora synon. obtusifolia synon. toroides Galactomannan und b) Carrageenan, Agar und/oder Xanthan enthält.

2. Gelier- und Verdickungsmittel gemäss Anspruch 1, dadurch gekennzeichnet, dass es die Komponenten in einem Verhältnis von 10-90 Gewichsteile a) zu 90-10 Gewichsteile b) enthält.

3. Gelier- und Verdickungsmittel gemäss Anspruch 2, dadurch gekennzeichnet, dass es die Komponenten in einem Verhältnis von 40-60 Gewichtsteile a) zu 60-40 Gewichtsteile b) enthält.

4. Gelier- und Verdickungsmittel gemäss einem der vorgehenden Ansprüche, dadurch gekennzeichnet, dass es Kalium-, Calcium- und/oder Ammoniumionen enthält.

5. Gelier- und Verdickungsmittel gemäss Anspruch 4, dadurch gekennzeichnet, dass es 1-50, vorzugsweise 10-40 Gew.-% Kaliumchlorid bezogen auf das Gewicht der Komponente b) enthält.

6. Gelier- und Verdickungsmittel gemäss einem der Ansprüche 1-5 in Form einer pulverförmigen Mischung.

7. Gelier- und Verdickungsmittel gemäss einem der Ansprüche 1-5 in Form eines wässerigen Gels.

8. Verwendung eines Gelier- und Verdickungsmittels gemäss einem der Ansprüche 1-7 bei der Verarbeitung von Lebensmitteln und Futtermitteln und für pharmazeutische und kosmetische Zwecke.

9. Verwendung eines Gelier- und Verdickungsmittels gemäss einem der Ansprüche 1-7 als Flokkungs-, Absetz- und Filterhilfsmittel sowie als Wasserretentionsmittel.

10. Verwendung eines Gelier- und Verdikkungsmittels gemäss einem der Ansprüche 1-7 als Verdickungsmittel für textile Anwendungen, für Sprengstofformulierungen und bei Erdöl- und Wasserbohrungen.

11. Verwendung von Cassia-tora synon. obtusifolia synon. toroides Galactomannan zusammen mit Carrageenan, Agar und/oder Xanthan zur Erzeugung eines synergistischen Gelier- und/oder Verdickungseffektes.

### Patentansprüche für folgenden Vertragsstaat: AT

1. Verfahren zur Herstellung eines Gelier- und Verdickungsmittels auf der Basis von Cassia-Galactomannannen, dadurch gekennzeichnet, dass man a) Cassia-tora synon. obtusifolia synon. toroides Galactomannan und b) Carrageenan, Agar und/oder Xanthan miteinander vermischt.

2. Verfahren gemäss Anspruch 1, dadurch gekennzeichnet, dass man die Komponenten in einem Verhältnis von 10-90 Gewichsteile a) zu 90-10 Gewichsteile b) einsetzt.

3. Verfahren gemäss Anspruch 2, dadurch gekennzeichnet, dass man die Komponenten in einem Verhältnis von 40-60 Gewichtsteile a) zu 60-40 Gewichtsteile b) einsetzt.

4. Verfahren gemäss einem der vorgehenden Ansprüche, dadurch gekennzeichnet, dass man Kalium-, Calcium- und/oder Ammoniumionen zusetzt.

5. Verfahren gemäss Anspruch 4, dadurch gekennzeichnet, dass man 1-50, vorzugsweise 10-40 Gew.-% Kaliumchlorid bezogen auf das Gewicht der Komponente b) zusetzt.

6. Verfahren gemäss einem der Ansprüche 1-5, dadurch gekennzeichnet, dass man die Komponenten in Pulverform einsetzt.

7. Verfahren gemässe einem der Ansprüche 1-5, dadurch gekennzeichnet, dass man die Komponenten zu einem wässerigen Gel verarbeitet.

8. Verwendung eines Gelier- und Verdickungsmittels hergestellt gemäss einem der Ansprüche 1-7 bei der Verarbeitung von Lebensmitteln und Futtermitteln und zur Herstellung von pharmazeutischen und kosmetischen Produkten.

9. Verwendung eines Gelier- und Verdickungsmittels hergestellt gemäss einem der Ansprüche 1-7 als Flockungs-, Absetz- und Filterhilfsmittel sowie als Wasserretentionsmittel.

10. Verwendung eines Gelier- und Verdikkungsmittels hergestellt gemäss einem der Ansprüche 1-7 als Verdickungsmittel für textile Anwendungen, für Sprengstofformulierungen und bei Erdöl- und Wasserbohrungen.

11. Verwendung von Cassia-tora synon. obtusifolia synon. toroides Galactomannan zusammen mit Carrageenan, Agar und/oder Xanthan zur Erzeugung eines synergistischen Gelier- und/oder Verdickungseffektes.


## Claims

### Claims for the following Contracting States : BE, CH, DE, FR, GB, IT, LI, LU, NL, SE

1. Jellying and thickening agent on the basis of Cassia galactomannans, characterized in that it contains a synergistic mixture of a) Cassia tora synon. obtusifolia synon. toroides galactomannans and b) carrageenan, agar and/or xanthane.

2. Jellying and thickening agent according to claim 1, characterized in that it contains the components at a ratio of 10-90 parts by weight a) to 90-10 parts by weight b).

3. Jellying and thickening agent according to claim 2, characterized in that it contains the components at a ratio of 40-60 parts by weight a) to 60-40 parts by weight b).

4. Jellying and thickening agent according to one of the previous claims, characterized in that it contains potassium ions, calcium ions and/or ammonium ions.

5. Jellying and thickening agent acccording to claim 4, characterized in that it contains 1-50, preferably 10-40 weight percent potassium chloride related to the weight of the component b).

6. Jellying and thickening agent according to one of claims 1-5, in the form of a pulverulent mixture.

7. Jellying and thickening agent according to one of claims 1-5, in the form of an aqueous gel.

8. Use of a jellying and thickening agent according to one of claims 1-7 in the processing of foodstuffs and fodder and for pharmaceutical and cosmetic purposes.

9. Use of a jellying and thickening agent according to one of claims 1-7 as floculation, setting and filtering auxiliary agents or waterretention agents.

10. Use of a jellying and thickening agent according to one of claims 1-7 as thickening agent for textile applications, for explosive formulations and oil and water drilling.

11. Use of Cassia tora synon.obtusifolia synon. toroides galactomannan together with carrageenan, agar and/or xanthane for the production of a synergistic jellying and/or thickening effect.

### Claims for the following Contracting States : AT

1. Process for the production of a jellying and thickening agent on the basis of Cassia galactomannans, characterized in that a) Cassia tora synon. obtusifolia synon. toroides galactomannan and b) carrageenan, agar and/or xanthane are mixed with each other.

2. Process according to claim 1, characterized in that the components are employed at a ratio of 10-90 parts by weight a) to 90-10 parts by weight b).

3. Process according to claim 2, characterized in that the components are employed at a ratio of 40-60 parts by weight a) to 60-40 parts by weight b).

4. Process according to one of the previous claims, characterized in that potassium ions, calcium ions and/or ammonium ions are added.

5. Process according to claim 4, characterized in that 1-50, preferably 10-40 weight percent potassium chloride related to the weight of component b) are added.

6. Process according to one of claims 1-5, characterized in that the components are used in pulverized form.

7. Process according to one of claims 1-5, characterized in that the components are processed to an aqueous gel.

8. Use of a jellying and thickening agent produced in accordance with one of claims 1-7 in the processing of foodstuffs and fodder or for the production of pharmaceutical and cosmetic products.

9. Use of a jellying and thickening agent produced in accordance with one of claims 1-7 as floculation, setting or filtering auxiliary agents as well as water-retention agents.

10. Use of a jellying and thickening agent produced in accordance with one of claims 1-7 as thickening agent for textile applications, for explosive formulation and in oil and water drilling.

11. Use of Cassia tora synon. obtusifolia synon. toroides galactomannan together with carrageenan, agar and/or xanthane for the production of a synergistic jellying and/or thickening effect.

## Revendications

### Revendications pour les Etats contractants suivants : BE, CH, DE, FR, GB, IT, LI, LU, NL, SE

1. Gélifiants et épaississants à base de galactomannanes de cassia, caractérisés par le fait qu'ils contiennent un mélange synergique a) de galactomannane de cassia tora synon. obtusifolia synon. toroides et b) de carraghénane, d'agar-agar et/ou de xanthane.

2. Gélifiants et épaississants selon la revendication 1, caractérisés par le fait qu'ils contiennent les constituants dans un rapport de 10-90 parties en poids de a) pour 90-10 parties en poids de b).

3. Gélifiants et épaississants selon la revendication 2, caractérisés par le fait qu'ils contiennent les constituants dans un rapport de 40-60 parties en poids de a) pour 60-40 parties en poids de b).

4. Gélifiants et épaississants selon l'une des revendications précédentes, caractérisés par le fait qu'ils contiennent des ions potassium, calcium et/ou ammonium.

5. Gélifiants et épaississants selon la revendication 4, caractérisés par le fait qu'ils contiennent 1-50, de préférence 10-40 % en poids de chlorure de potassium par rapport au poids du constituant b).

6. Gélifiants et épaississants selon l'une des revendications 1-5, sous la forme d'un mélange pulvérulent.

7. Gélifiants et épaississants selon l'une des revendications 1-5 sous la forme d'un gel aqueux.

8. Utilisation d'un gélifiant et d'un épaississant selon l'une des revendications 1-7, dans le traitement des aliments pour l'homme et les animaux et à des fins pharmaceutiques et cosmétiques.

9. Utilisation d'un gélifiant et d'un épaississant selon l'une des revendications 1-7 comme agent floculant, agent de décantation et agent de filtration ainsi que comme agent de rétention d'eau.

10. Utilisation d'un gélifiant et d'un épaississant selon l'une des revendications 1-7 comme épaississant pour des applications textiles, pour des formules d'explosifs, et pour des forages pour la recherche du pétrole et de l'eau.

11. Utilisation du galactomannane de cassia tora synon. obtusifolia synon. toroides en même temps que du carraghénane, de l'agar-agar et/ou du xanthane pour la production d'un effet de gélification et/ou d'épaississant synergique.

### Revendications pour l'Etat contractant suivant : AT

1. Procédé de préparation d'un agent de gélification et d'épaississement à base de galactomannanes de cassia, caractérisé par le fait qu'on mélange a) du galactomannane de cassia tora synon. obtusifolia synon. toroides et b) du carraghénane, de l'agar-agar et/ou du xanthane.

2. Procédé selon la revendication 1, caractérisé par le fait qu'on utilise les constituants dans un rapport de 10 à 90 parties en poids de a) pour 90-10 parties en poids de b).

3. Procédé selon la revendication 2, caractérisé par le fait qu'on utilise les constituants dans un rapport de 40-60 parties en poids de a) pour 60-40 parties en poids de b).

4. Procédé selon l'une des revendications précédentes, caractérisé par le fait qu'on ajoute des ions potassium, calcium et/ou ammonium.

5. Procédé selon la revendication 4, caractérisé par le fait qu'on ajoute 1-50, de préférence 10-40 % en poids de chlorure de potassium par rapport au poids du constituant b).

6. Procédé selon l'une des revendications 1-5, caractérisé par le fait qu'on utilise les constituants sous forme de poudre.

7. Procédé selon l'une des revendications 1-5, caractérisé par le fait qu'on transforme les constituants en un gel aqueux.

8. Utilisation d'un gélifiant et d'un épaississant préparé selon l'une des revendications 1-7, lors du traitement d'aliments pour l'homme et des animaux, et pour la préparation de produits pharmaceutiques et cosmétiques.

9. Utilisation d'un gélifiant et d'un épaississant préparé selon l'une des revendications 1-7 comme agent de floculation, agent de décantation et agent de filtration, ainsi que comme agent de rétention d'eau.

10. Utilisation d'un gélifiant et d'un épaississant préparé selon l'une des revendications 1-7 comme épaississant pour des applications textiles, pour des formules d'explosif et dans des forages pour la recherche de pétrole et d'eau.

11. Utilisation de galactomannane de cassia tora synon. obtusifolia synon. toroides en même temps que de carraghénane, d'agar-agar et/ou de xanthane pour la production d'un effet gélifiant et/ou épaississant synergique.